(19) Europäisches Patentamt
European Patent Office
Office européen des brevets

(11) **EP 4 721 672 A1**

(12) **EUROPEAN PATENT APPLICATION**
published in accordance with Art. 153(4) EPC

(43) Date of publication:
08.04.2026 Bulletin 2026/15

(51) International Patent Classification (IPC):
**A61B 8/00** (2006.01)

(21) Application number: 24814477.6

(22) Date of filing: 29.05.2024

(86) International application number:
PCT/CN2024/096044

(87) International publication number:
WO 2024/245280 (05.12.2024 Gazette 2024/49)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 30.05.2023 CN 202310626184

(71) Applicant: Wuxi Hisky Medical Technologies Co.,
Ltd.
Wuxi, Jiangsu 214000 (CN)

(72) Inventors:
• HE, Qiong
Wuxi, Jiangsu 214000 (CN)
• SHAO, Jinhua
Wuxi, Jiangsu 214000 (CN)
• SUN, Jin
Wuxi, Jiangsu 214000 (CN)

(74) Representative: Elzaburu S.L.P.
Paseo de la Castellana 259C
Torre de Cristal, planta 28
28046 Madrid (ES)

(54) **ELASTOGRAPHY METHOD, APPARATUS AND DEVICE, AND STORAGE MEDIUM**

(57) The present application relates to the field of elastography technologies, and in particular, to an elastography method, apparatus and device, and a storage medium. The method includes: acquiring an elastography area of a to-be-detected tissue and a target pressure range of the to-be-detected tissue; determining a detection position where an elasticity detection probe is located when performing elasticity detection in the elastography area; performing the elasticity detection in the elastography area for a plurality of times based on the detection position and a plurality of pressure values within the target pressure range, to acquire a plurality of elastography results; and fusing the plurality of elastography results to determine a target elastography result of the to-be-detected tissue. The impacts of different pressures on measurement result are taken into account, and the elasticity detection is performed under different pressures to acquire the plurality of elasticity imaging results, and the to-be-detected tissue is comprehensively evaluated based on the plurality of elasticity imaging results, so that a more accurate measurement result can be obtained.

Acquiring an elastography area of a to-be-detected tissue and a target pressure range of the to-be-detected tissue — S11

Determining a detection position where an elasticity detection probe is located when performing elasticity detection in the elastography area — S12

Performing the elasticity detection in the elastography area for a plurality of times based on the detection position and a plurality of pressure values within the target pressure range, to obtain a plurality of elastography results — S13

Fusing the plurality of elastography results to determine a target elastography result of the to-be-detected tissue — S14

FIG. 1

EP 4 721 672 A1

## Description

This application claims priority to Chinese Patent Application No. 202310626184.1, filed with the China National Intellectual Property Administration on May 30, 2023 and entitled "ELASTOGRAPHY METHOD, APPARATUS AND DEVICE, AND STORAGE MEDIUM", which is hereby incorporated by reference in its entirety.

## TECHNICAL FIELD

The present application relates to the field of elastography technologies, and in particular, to an elastography method, apparatus and device, and a storage medium.

## BACKGROUND

The elastography technology has been widely applied in various fields, and in clinical application, the acquired elasticity information of a biological material is usually converted into a pseudo-color image which is customary to a doctor, so that the doctor can discriminate the material mechanical properties of a tissue through the pseudo-color image, and then determine an elasticity detection result of an organism according to the soft and hard conditions of the tissue.

When the elastography technology is applied in clinic, the doctor holds an elasticity detection probe to perform elastography scanning, to obtain an elastography result. However, when the elastography is performed on the same tissue each time, the acquired elasticity detection result is relatively singular, leading to low accuracy of the elastography result.

## SUMMARY

In view of this, the embodiments of the present application provide an elastography method, apparatus and device, and a storage medium, to solve the problem of low accuracy of the elastography result.

According to a first aspect, an embodiment of the present application provides an elastography method, including:

> acquiring an elastography area of a to-be-detected tissue and a target pressure range of the to-be-detected tissue;
> determining a detection position where an elasticity detection probe is located when performing elasticity detection in the elastography area;
> performing the elasticity detection in the elastography area for a plurality of times based on the detection position and a plurality of pressure values within the target pressure range, to obtain a plurality of elastography results; and
> fusing the plurality of elastography results to determine a target elastography result of the to-be-detected tissue.

In the elastography method provided in the embodiments of the present application, the impacts of different pressures on a measurement result are taken into account, and the elastography is performed multiple times on the to-be-detected tissue through the plurality of pressure values within the target pressure range, so as to obtain the plurality of elastography results. Since the pressure values during the plurality of elasticity measurements are all within the target pressure range, the reliability of the plurality of elastography results can be ensured. In addition, the plurality of elastography results are used to perform a comprehensive evaluation on the to-be-detected tissue, so that a more accurate, more stable and more comprehensive measurement result can be obtained.

In some embodiments, the performing the elasticity detection in the elastography area for the plurality of times based on the detection position and the plurality of pressure values within the target pressure range, to obtain the plurality of elastography results includes:

> acquiring a pressure value currently applied to the elastography area when the elasticity detection probe performs ultrasonic scanning at the detection position, to obtain a current pressure value; and
> sequentially adjusting the current pressure value to each pressure value among the plurality of pressure values, and performing the elasticity detection in the elastography area based on the detection position and the adjusted pressure value each time, to obtain the plurality of elastography results.

In the elastography method provided in the embodiments of the present application, the current pressure value is adjusted to each pressure value among the plurality of pressure values sequentially, to ensure that the pressure applied for each elasticity detection is within the target pressure range, thereby improving the efficiency of elastography and the accuracy of the measurement result.

In some embodiments, the acquiring the current pressure value includes:
acquiring the current pressure value through a pressure detection unit corresponding to the elasticity detection probe, where the pressure detection unit is configured to detect a pressure applied by the elasticity detection probe to the elastography area.

In the elastography method provided in the embodiments of the present application, the pressure detection unit is used to measure the pressure applied by the elastography probe to the elastography area, to obtain the current pressure value, thereby realizing real-time detection of the pressure applied by the elastography probe to the elastography area.

In some embodiments, the performing the elasticity detection in the elastography area based on the detection position and the adjusted pressure value each time, to obtain the plurality of elastography results includes:

performing, based on the detection position, the elasticity detection in the elastography area respectively by using the adjusted pressure value each time, to obtain a group velocity as well as a phase velocity of a shear wave at different pressure values; and

determining an elastography result corresponding to each pressure value based on a group velocity as well as a phase velocity of the shear wave at different pressure values.

In some embodiments, the fusing the plurality of elastography results to determine the target elastography result of the to-be-detected tissue includes:

determining pressure index information based on the group velocity as well as the phase velocity of the shear wave at different pressure values; and

fusing the plurality of elastography results based on the pressure index information, to determine the target elastography result.

In the elastography method provided in the embodiments of the present application, not only the impacts brought about by different pressures when the elastography is performed on the tissue are taken into account, but also more abundant and more accurate parameters of the tissue can be obtained. That is, by using the plurality of parameters, the elastography result can be determined more accurately, thereby achieving a more accurate evaluation of the to-be-detected tissue.

In some embodiments, the performing the elasticity detection in the elastography area for the plurality of times based on the detection position and the plurality of pressure values within the target pressure range, to obtain the plurality of elastography results includes:

acquiring a target detection direction corresponding to the elastography area, where the target detection direction is a normal vector direction of the detection position;

acquiring a current detection direction of the elasticity detection probe at the detection position;

controlling the elasticity detection probe to move based on a difference between the current detection direction and the target detection direction, to adjust the current detection direction to the target detection direction; and

performing the elasticity detection in the elastography area for the plurality of times according to the adjusted current detection direction and the plurality of pressure values within the target pressure range, to obtain the plurality of elastography results.

In the elastography method provided in the embodiments of the present application, the difference between the current detection direction and the target detection direction is used to automatically control the movement of the elastography probe, to realize automatic control of the elastography probe, thereby achieving the elasticity detection for the plurality of times and ensuring the accuracy of the plurality of acquired elastography results.

In some embodiments, the performing the elasticity detection in the elastography area for the plurality of times based on the detection position and the plurality of pressure values within the target pressure range, to obtain the plurality of elastography results includes:

acquiring a target detection direction corresponding to the elastography area, where the target detection direction is a normal vector direction of the detection position;

acquiring a current detection direction of the elasticity detection probe at the detection position;

controlling the elasticity detection probe to move by a corresponding angle based on a difference between the current detection direction and the target detection direction, to adjust the current detection direction to the target detection direction;

acquiring a pressure value applied to the elastography area when the elasticity detection probe performs ultrasonic scanning at the detection position along the adjusted current detection direction to obtain a current pressure value; and

sequentially adjusting the current pressure value to each pressure value among the plurality of pressure values, and performing the elasticity detection in the elastography area by using the adjusted pressure value each time, to obtain the plurality of elastography results.

In some embodiments, the performing the elasticity detection in the elastography area for the plurality of times based on the detection position and the plurality of pressure values within the target pressure range, to obtain the plurality of elastography results includes:

determining a normal vector direction of the detection position;
acquiring a pressure value applied to the elastography area when the elasticity detection probe performs ultrasonic scanning at the detection position along the normal vector direction to obtain a current pressure value; and
sequentially adjusting the current pressure value to each pressure value among the plurality of pressure values, and performing the elasticity detection in the elastography area by using the adjusted pressure value each time, to obtain the plurality of elastography results.

According to a second aspect, an embodiment of the present application further provides an elastography apparatus, including:

an acquisition module, configured to acquire an elastography area of a to-be-detected tissue and a target pressure range of the to-be-detected tissue;
a first determination module, configured to determine a detection position where an elasticity detection probe is located when performing elasticity detection in an elastography area;
an imaging module, configured to perform the elasticity detection in the elastography area for a plurality of times based on the detection position and a plurality of pressure values within the target pressure range, to obtain a plurality of elastography results; and
a second determination module, configured to fuse the plurality of elastography results to determine a target elastography result of the to-be-detected tissue.

According to a third aspect, an embodiment of the present application provides an elastography device, including: a memory and a processor, where the memory and the processor are in communication connection with each other, the memory stores computer instructions, and the processor executes the computer instructions, to implement the elastography method in the first aspect or any one of the embodiments of the first aspect.

According to a fourth aspect, an embodiment of the present application provides a computer-readable storage medium, where the computer-readable storage medium stores computer instructions, and the computer instructions are used to enable a computer to implement the elastography method in the first aspect or any one of the embodiments of the first aspect.

It should be noted that the corresponding beneficial effects of the elastography apparatus, the elastography device, and the readable storage medium provided in the embodiments of the present application may be referred to the description of corresponding beneficial effects of the elastography method, which will not be repeated herein.

## BRIEF DESCRIPTION OF THE DRAWINGS

In order to illustrate the technical solutions in the embodiments of the present application or in the prior art more clearly, a brief description will be given to the accompanying drawings required for the description of the embodiments or the prior art. Obviously, the accompanying drawings in the following description are merely some embodiments of the present application, and for those ordinary skilled in the art, other drawings can also be acquired from these drawings without creative efforts.

FIG. 1 is a flowchart of an elastography method according to an embodiment of the present application.
FIG. 2 is a flow chart of an elastography method according to another embodiment of the present application.
FIG. 3 is a flowchart of an elastography method according to yet another embodiment of the present application.
FIG. 4 is a structural block diagram of an elastography apparatus according to an embodiment of the present application.
FIG. 5 is a schematic diagram of a hardware structure of an elastography device according to an embodiment of the present application.

## DESCRIPTION OF EMBODIMENTS

In order to make the objectives, technical solutions, and advantages of the embodiments of the present application clearer, the technical solutions in the embodiments of the present application will be clearly and completely described in the below with reference to the accompanying drawings in the embodiments of the present application. Obviously, the described embodiments are merely some rather than all of the embodiments of the present application. Based on the embodiments of the present application, all other embodiments made by those ordinary skilled in the art without creative efforts shall fall within the protection scope of the present application.

According to the embodiments of the present application, an elastography method embodiment is provided. It should be noted that the steps shown in the flowchart of the accompanying drawings can be executed in a computer system such as a set of computer-executable instructions, and although the logic order is shown in the flowchart, in some cases, the shown or described steps can be executed in an order different from that described here.

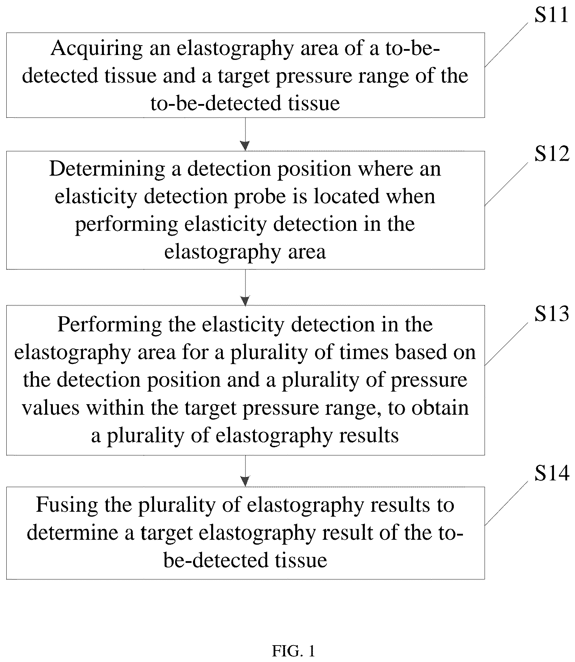

In this embodiment, an elastography method is provided, which can be applied in an elastography device. FIG. 1 is a flowchart of the elastography method according to an embodiment of the present application. As shown in FIG. 1, the flow includes the following steps.

S11, acquiring an elastography area of a to-be-detected tissue and a target pressure range of the to-be-detected tissue.

An ultrasonic image can be acquired by performing ultrasonic scanning firstly, and then a region of interest on the ultrasonic image is identified to determine the elastography area. Alternatively, a tissue structural diagram is displayed on an imaging interface, and when the ultrasonic imaging is performed on the to-be-detected tissue, a position corresponding to the probe in the tissue structural diagram is identified based on a contact position between the probe and the to-be-detected tissue, to give the users with guidance on the elastography area, thereby locating the elastography area quickly.

The target pressure range of the to-be-detected tissue may be specified by the users, and may also be set in the elastography device. For example, there are corresponding relationships between different tissues and pressure ranges provided in the elastography device. Based on this, the target pressure range of the to-be-detected tissue can be obtained through the corresponding relationships.

The target pressure ranges of different tissues to-be-detected may be different, for example, the target pressure range of a breast tissue is 0.002-5N, and the target pressure range of a liver part is 0.005-15N.

S12, determining a detection position where an elasticity detection probe is located when performing elasticity detection in the elastography area.

When starting the elasticity detection, the position where the elasticity detection probe is located, that is, the detection position may have a plurality of candidate detection positions in the elastography area. The detection position in this embodiment refers to a position where the probe starts to perform ultrasonic scanning when the elasticity detection is performed. The position may be any one of the plurality of candidate detection positions, and may also be a position with a best detection effect. The detection position may also be referred to as a spatial location.

S13, performing the elasticity detection in the elastography area for a plurality of times based on the detection position and a plurality of pressure values within the target pressure range, to obtain a plurality of elastography results.

The target pressure range is an optimum pressure range applied by the elasticity detection probe to the elastography area. In the target pressure range, on the basis of the determined spatial position, the elasticity detection is performed on the elastography area by using each pressure value among the plurality of pressure values, to obtain an elastography result that corresponds one-to-one with each pressure value, thereby obtaining the plurality of elastography results. A quantity of the acquired plurality of pressure values can be acquired according to requirements, and the plurality of pressure values can be distributed at equal intervals or distributed at unequal intervals, which is not limited herein, and can be specifically set according to actual requirements.

The pressure applied by the elasticity detection probe to the elastography area can be measured by a pressure sensor provided in the elasticity detection probe; may also be acquired by using an additional pressure detection unit, or may be acquired by other means, which is not limited herein. The pressure applied by the elasticity detection probe to the elastography area is also a contact pressure between the elasticity detection probe and the elastography area, and thus can also be referred to as a contact pressure.

S14, fusing the plurality of elastography results to determine a target elastography result of the to-be-detected tissue.

The elastography results correspond one-to-one with the pressure values, and there are also differences between the elastography results of different pressure values. On this basis, the differences between the elastography results of different pressure values can be used to obtain mechanical properties of the to-be-detected tissue under different pressures. On this basis, combined with the plurality of elastography results, the measurement result of the to-be-detected tissue, i.e., the target elastography result, can be determined more accurately.

In the elastography method provided in this embodiment, the elasticity detection is performed under different pressures to obtain the plurality of elastography results, and a comprehensive evaluation on the to-be-detected tissue is performed based on the plurality of elastography results to obtain a more accurate measurement result. Since the pressure values during the plurality of elasticity measurements are all within the target pressure range, the reliability of the plurality of elastography results can be ensured, and the accuracy of the finally obtained measurement result can be ensured. In this way, the impacts of different pressures on the measurement result are taken into account, and a plurality of measurement results of the pressure parameters are introduced to perform a comprehensive evaluation on the tissue, so as to evaluate the mechanical properties of the tissue more accurately and obtain a more accurate measurement result.

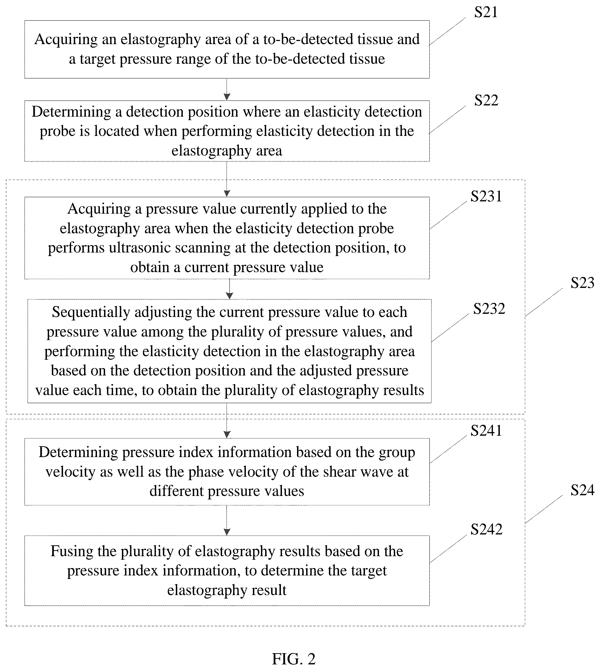

In this embodiment, an elastography method is provided, which can be applied to an elastography device. FIG. 2 is a flowchart of the elastography method according to another embodiment of the present application. As shown in FIG. 2, the flow includes the following steps.

S21, acquiring an elastography area of a to-be-detected tissue and a target pressure range of the to-be-detected tissue.

For details, please refer to S11 in the embodiment shown in FIG. 1, which will not be repeated herein.

S22, determining a detection position where an elasticity detection probe is located when performing elasticity detection in the elastography area.

For details, please refer to S12 in the embodiment shown in FIG. 1, which will not be repeated herein.

S23, performing the elasticity detection in the elastography area for a plurality of times based on the detection position and a plurality of pressure values within the target pressure range, to obtain a plurality of elastography results.

Specifically, the above-mentioned S23 includes the following steps.

S231, acquiring a pressure value currently applied to the elastography area when the elasticity detection probe performs ultrasonic scanning at the detection position, to obtain a current pressure value.

When the elasticity detection probe performs ultrasonic scanning at the detection position, the current pressure value applied to the elastography area is acquired from a pressure detection unit corresponding to the elasticity detection probe, where the pressure detection unit is configured to detect the pressure (i.e., contact pressure) applied by the elasticity detection probe to the elastography area, and the pressure detection unit is provided inside the elasticity detection probe or is adapted to the elasticity detection probe.

When the pressure detection unit is provided inside the elasticity detection probe, the pressure detection unit may be a built-in pressure sensor; when the pressure detection unit is adapted to the elasticity detection probe, the pressure detection unit is placed between the elasticity detection probe and the elastography area, to measure the indirect contact pressure between the elasticity detection probe and the elastography area during performing elastography. The specific structural details of the pressure detection unit are not limited herein, as long as it can measure the contact pressure between the elasticity detection probe and the elastography area.

The pressure detection unit is used to measure the pressure applied by the elasticity detection probe to the elastography area, to obtain the current pressure value, thereby realizing real-time detection of the pressure applied by the elasticity detection probe to the elastography area.

S232, sequentially adjusting the current pressure value to each pressure value among the plurality of pressure values, and performing the elasticity detection in the elastography area based on the detection position and the adjusted pressure value each time, to obtain the plurality of elastography results.

The adjustment of the current pressure value can be achieved by displaying a relationship between the current pressure value and the target pressure range on an imaging interface, to sequentially adjust the magnitude of the current pressure value to each of the plurality of pressure values within the target pressure range.

The imaging interface is an interface for elastography, but in this embodiment, it is not limited to an interface, but is a general term of a type of interface, that is, the interface used for elastography. After the current pressure value is obtained, the current pressure value is compared with the target pressure range, to determine a magnitude relationship between the current pressure value and the target pressure range, and display the magnitude relationship on the imaging interface.

For example, if the current pressure value is greater than a maximum value of the target pressure range, it is indicated by a red mark; if the current pressure value is within the target pressure range, it is indicated by a green mark; and when the current pressure value is less than a minimum value of the target pressure range, it is indicated by a yellow mark.

It should be noted that, the described colors are only examples and does not limit the protection scope of the present application, and can be specifically set according to actual requirements, as long as they can represent the relationship between the current pressure value and the target pressure range on the imaging interface.

Each time the elastography is performed, the current pressure value can be adjusted through the above-mentioned manner, to adjust the current pressure value to the plurality of pressure values within the target pressure range respectively.

Based on the detection position, the elasticity detection is respectively performed in the elastography area by using each of the plurality of pressure values, and after each elasticity detection, an elastography result can be obtained, and accordingly, the plurality of elastography results can be obtained by using the plurality of pressure values.

In the imaging method provided in this embodiment, the current pressure value is adjusted to each pressure value among the plurality of pressure values before the elasticity detection, and then the elasticity detection is performed, so that it can be ensured that an elastography result corresponding to the desired pressure value can be obtained, thereby improving the accuracy of the finally obtained elastography result and the efficiency of elastography.

In some embodiments, the performing the elasticity detection in the elastography area based on the detection position and the adjusted pressure value each time, to obtain the plurality of elastography results, as shown in the above-mentioned S32, includes:

(1) performing, based on the detection position, the elasticity detection in the elastography area respectively by using the adjusted pressure value each time, to obtain a group velocity as well as a phase velocity of a shear wave at different pressure values;
(2) determining the elastography result corresponding to each pressure value based on the group velocity as well as the phase velocity of the shear wave at different pressure values.

Based on the detection position, the elasticity detection is performed by using the plurality of pressure values, to obtain the group velocity as well as the phase velocity of the shear wave, where the group velocity is a propagation velocity of a wave-front of the shear wave in the tissue, and the phase velocity is a propagation velocity of different frequency components of the shear wave in the tissue. After obtaining the group velocity and the phase velocity, the elasticity information of the to-be-detected tissue can be determined by analyzing the group velocity and the phase velocity, and the elasticity information is the elastography result.

S24, fusing the plurality of elastography results to determine a target elastography result of the to-be-detected tissue.

Specifically, the above-mentioned S24 includes the following steps.

S241, determining pressure index information based on the group velocity as well as the phase velocity of the shear wave at different pressure values.

The elastography result and the pressure index information corresponding to each pressure value can be calculated according to the following formula:

$$V(t, f) = f(V0(t), E(t), \mu(t), b, f, \sigma, \rho, E0, P(t));$$

where, $V0(t)$ is the group velocity, $V(t, f)$ is the phase velocity, $E(t)$ is a Young's modulus, $\mu(t)$ is a viscous modulus, $b$ is the pressure index information, $\rho$ is a density, $\sigma$ is a Poisson's ratio, with a value of 0.5, $E0$ is a volume modulus of the tissue, $P(t)$ is the pressure value, $f$ is a frequency of the shear wave, $t$ has a value of 1-N, and N is a quantity of the plurality of pressure values.

S242, fusing the plurality of elastography results based on the pressure index information, to determine the target elastography result.

After obtaining the plurality of elastography results, a further analysis is conducted by combining the pressure index information and the elastography result corresponding to the pressure value related to the obtained pressure index information, that is, the elastography result corresponding to the pressure involved when determining the pressure index information is analyzed by using the pressure index information, to further determine the target elastography result.

Specifically, according to the above formula, the elastography results under different pressures can be obtained, and then the target elastography result is determined based on a frequency-dispersion curve fitting method.

In the imaging method provided in this embodiment, not only the impacts brought about by different pressures when the elastography is performed on the tissue are taken into account, but also more abundant and more accurate parameters of the tissue can be obtained, and the mechanical characteristics of the tissue can further be evaluated more accurately by introducing the plurality of parameters, that is, the elastography result can be determined more accurately, thereby achieving a more accurate evaluation of the to-be-detected tissue.

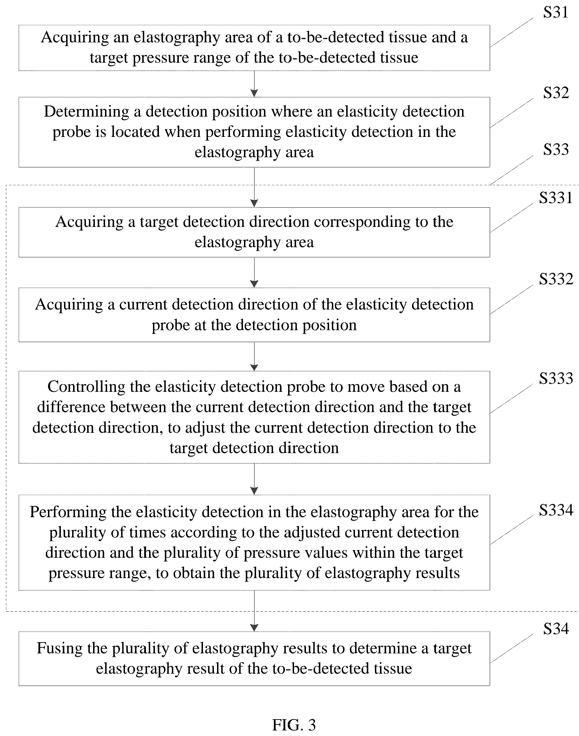

In this embodiment, an elastography method is provided, which can be applied to an elastography device. FIG. 3 is a flowchart of an elastography method according to yet another embodiment of the present application. As shown in FIG. 3, the flow includes the following steps.

S31, acquiring an elastography area of a to-be-detected tissue and a target pressure range of the to-be-detected tissue.

For details, please refer to S11 in the embodiment shown in FIG. 1, which will not be repeated herein.

S32, determining a detection position where an elasticity detection probe is located when performing elasticity detection in the elastography area.

For details, please refer to S12 in the embodiment shown in FIG. 1, which will not be repeated herein.

S33, performing the elasticity detection in the elastography area for a plurality of times based on the detection position and a plurality of pressure values within the target pressure range, to obtain a plurality of elastography results.

In some embodiments, the above-mentioned S33 includes the following steps.

S331, acquiring a target detection direction corresponding to the elastography area.

The target detection direction is a normal vector direction of the detection position.

The target detection direction is determined according to the detection position, and specifically is a normal vector direction of the detection position. For example, the surface shape distribution of the to-be-detected tissue is obtained according to the scanning result of ultrasonic imaging and the position information of the detection position of the probe, and the normal vector direction, i.e., the target detection direction, at the detection position can be determined according to the surface shape distribution and the position information of the elasticity detection probe. Specifically, a detection position set $(X_n, Y_n, Z_n)$ on the surface of the elastography area is obtained according to the change information of the detection position of the elasticity detection probe; and then the normal vector direction of the detection position is determined according to the spatial three-dimensional coordinates, to determine the normal vector direction of the detection position.

S332, acquiring a current detection direction of the elasticity detection probe at the detection position.

The probe may have different angle orientations at the detection position, and the different angle orientations correspond to different detection directions, and therefore, it is necessary to obtain a current angle orientation of the elasticity detection probe at the detection position.

S333, controlling the elasticity detection probe to move based on a difference between the current detection direction and the target detection direction, to adjust the current detection direction to the target detection direction.

By comparing the difference between the current detection direction and the target detection direction, the movement amount of the elasticity detection probe is determined, and then the elasticity detection probe is controlled to move, to adjust the current detection direction to the target detection direction. The movement of the elasticity detection probe includes, but is not limited to, an angle movement, etc., which is specifically set according to actual requirements.

S334, performing the elasticity detection in the elastography area for the plurality of times according to the adjusted current detection direction and the plurality of pressure values within the target pressure range, to obtain the plurality of elastography results.

After the current detection direction is adjusted to the target detection direction, the elasticity detection is performed in the elastography area for the plurality of times along the adjusted current detection direction by using the plurality of pressure values within the target pressure range, to obtain the elastography results that corresponds one-to-one with each pressure value, thereby obtaining the plurality of elastography results.

In some embodiments, the above-mentioned S33 includes:

(1) acquiring a target detection direction corresponding to the elastography area, where the target detection direction is a normal vector direction of a detection position.
(2) acquiring a current detection direction of the elasticity detection probe at the detection position.
(3) controlling the elasticity detection probe to move based on a difference between the current detection direction and the target detection direction, to adjust the current detection direction to the target detection direction.
(4) acquiring a pressure value applied to the elastography area when the elasticity detection probe performs ultrasonic scanning at the detection position along the adjusted current detection direction, to obtain a current pressure value.
(5) sequentially adjusting the current pressure value to each pressure value among the plurality of pressure values, and performing the elasticity detection in the elastography area by using the adjusted pressure value each time, to obtain the plurality of elastography results.

The elasticity detection probe is connected through a mechanical arm, and by comparing the difference between the current detection direction and the target detection direction and controlling the elasticity detection probe to move a corresponding angle according to the difference, the current detection direction can be adjusted to the target detection direction automatically.

With regard to the method of acquiring the current pressure value and adjusting the current pressure value, please refer to the previous text and will not be repeated herein.

After adjusting to obtain each pressure value among the plurality of pressure values, the elasticity detection is performed in the elastography area along the target detection direction by using the adjusted pressure value each time, to obtain the plurality of elastography results.

In some embodiments, the above-mentioned S33 includes:

(1) determining a normal vector direction of the detection position.
(2) acquiring a pressure value applied to the elastography area when the elasticity detection probe performs ultrasonic scanning at the detection position along a normal vector direction, to obtain a current pressure value.
(3) sequentially adjusting the current pressure value to each pressure value among the plurality of pressure values, and performing the elasticity detection in the elastography area by using the adjusted pressure value each time, to obtain the plurality of elastography results.

Specifically, the detection position may be used to obtain its surface extending direction, and then a normal vector of the surface extending direction is calculated, to obtain the normal vector direction.

Specifically, a detection position set (Xn, Yn, Zn) on the surface of the elastography area may also be obtained according to the change information of the detection position of the ultrasonic probe; then the normal vector direction of the detection position is determined according to the spatial three-dimensional coordinates.

After determining the normal vector direction of the detection position, the elasticity detection probe is controlled to perform ultrasonic scanning at the detection position along the normal vector direction, the pressure value applied by the elasticity detection probe to the elastography area is detected to obtain the current pressure value, and then the current pressure value is sequentially adjusted to each pressure value among the plurality of pressure values, so that the elasticity detection can be performed for a plurality of times in the elastography area respectively by using the plurality of pressure values within the target pressure range, thereby acquiring an elastography result that corresponds one-to-one with each pressure value. In this embodiment, the elasticity detection probe is controlled to apply pressures of different sizes (for example, 3N, 5N, and 8N) to the elastography area of the to-be-detected tissue at the same angle and at the same detection position, and then the elasticity detection is performed for each pressure to obtain an elastography result corresponding to each pressure.

S34, fusing the plurality of elastography results to determine a target elastography result of the to-be-detected tissue.

For details, please refer to S24 in the embodiment shown in FIG. 2, which will not be repeated herein.

In the elastography method provided in this embodiment, the difference between the current detection direction and the target detection direction is used to automatically control the movement of the elastography probe, thereby realizing automatic control of the elastography probe, so as to realize the elasticity detection for the plurality of times and ensure the accuracy of the obtained plurality of elastography results.

It can be seen from the above that the present application provides a method for triggering the elastography of the to-be-detected tissue under different pressures and using the plurality of imaging results to evaluate the tissue comprehensively, thus enabling more accurate evaluation of the to-be-detected tissue.

In this embodiment, an elastography apparatus is also provided, which is configured to implement the above-mentioned embodiments and the preferred implementations, and those that have already been described will not be repeated herein. The term "module", as used hereinafter, is a combination of software and/or hardware capable of realizing a predetermined function. Although the apparatus described in the following embodiment is preferably implemented by software, implementation of hardware or a combination of software and hardware is also possible and conceived.

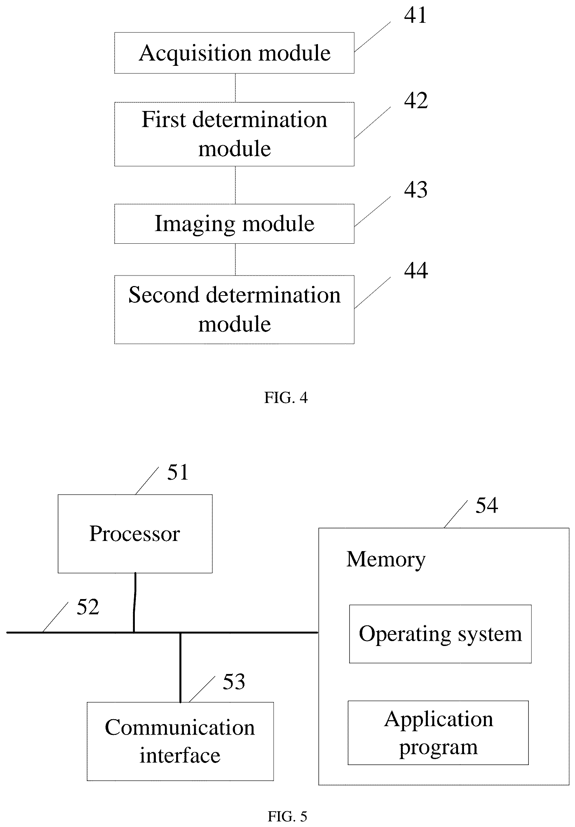

This embodiment provides an elastography apparatus, as shown in Fig. 4, including:

an acquisition module 41, configured to acquire an elastography area of a to-be-detected tissue and a target pressure range of the to-be-detected tissue;
a first determination module 42, configured to determine a detection position where an elasticity detection probe is located when performing elasticity detection in an elastography area;
an imaging module 43, configured to perform the elasticity detection in the elastography area for a plurality of times based on the detection position and a plurality of pressure values within the target pressure range, to obtain a plurality of elastography results; and
a second determination module 44, configured to fuse the plurality of elastography results to determine a target elastography result of the to-be-detected tissue.

In some embodiments, the imaging module 43 includes:

a first acquisition unit, configured to acquire a pressure value currently applied to the elastography area when the elasticity detection probe performs ultrasonic scanning at the detection position, to obtain a current pressure value; and
an imaging unit, configured to sequentially adjust the current pressure value to each pressure value among the plurality of pressure values, and perform the elasticity detection in the elastography area based on the detection position and the adjusted pressure value each time, to obtain the plurality of elastography results.

In some embodiments, the first acquisition unit includes:
a first acquisition sub-unit, configured to acquire the current pressure value through a pressure detection unit corresponding to the elasticity detection probe, where the pressure detection unit is configured to detect a pressure applied by the elasticity detection probe to the elastography area.

In some embodiments, the imaging unit includes:

a first obtaining sub-unit, configured to perform, based on the detection position, the elasticity detection in the elastography area respectively by using the adjusted pressure value each time, to obtain a group velocity as well as a phase velocity of a shear wave at different pressure values; and
a second obtaining sub-unit, configured to determine the elastography result corresponding to each pressure value based on the group velocity as well as the phase velocity of the shear wave at different pressure values.

In some embodiments, the second determination module 44 includes:

a first determination unit, configured to determine pressure index information based on the group velocity as well as the phase velocity of the shear wave at different pressure values; and
a second determination unit, configured to fuse a plurality of elastography results based on the pressure index information, to determine the target elastography result of the to-be-detected tissue.

In some embodiments, the imaging module 43 includes:

a second acquisition unit, configured to acquire a target detection direction corresponding to the elastography area, where the target detection direction is a normal vector direction of the detection position;
a third acquisition unit, configured to acquire a current detection direction of the elasticity detection probe at the detection position;
a first control unit, configured to control the elasticity detection probe to move based on a difference between the current detection direction and the target detection direction, to adjust the current detection direction to the target detection direction; and
a first detection unit, configured to perform the elasticity detection in the elastography area for the plurality of times according to the adjusted current detection direction and the plurality of pressure values within the target pressure range, to obtain the plurality of elastography results.

In some embodiments, the imaging module 43 includes:

a fourth acquisition unit, configured to acquire a target detection direction corresponding to the elastography area, where the target detection direction is a normal vector direction of the detection position;
a fifth acquisition unit, configured to obtain a current detection direction of the elasticity detection probe at the detection position;
a second control unit, configured to control the elasticity detection probe to move by a corresponding angle based on a difference between the current detection direction and the target detection direction, to adjust the current detection direction to the target detection direction;
a sixth acquisition unit, configured to acquire a pressure value applied to the elastography area when the elasticity detection probe performs ultrasonic scanning at the detection position along the adjusted current detection direction, to obtain a current pressure value; and
a second detection unit, configured to adjust the current pressure value to each pressure value among the plurality of pressure values, and perform the elasticity detection in the elastography area by using the adjusted pressure value each time, to obtain the plurality of elastography results.

In some embodiments, the imaging module 43 includes:

a third determination unit, configured to determine a normal vector direction of the detection position;
a seventh acquisition unit, configured to acquire a pressure value applied to the elastography area when the elasticity detection probe performs ultrasonic scanning at the detection position along the normal vector direction, to obtain a current pressure value; and
a third detection unit, configured to sequentially adjust the current pressure value to each pressure value among a plurality of pressure values, and perform the elasticity detection in the elastography area by using the adjusted pressure value each time, to obtain the plurality of elastography results.

The imaging apparatus in this embodiment is presented in the form of a functional unit, and the unit herein refers to an ASIC circuit, a processor and a memory that can execute one or more software or fixed programs, and/or other devices that can provide the foregoing functions.
Further functional descriptions of the above-mentioned modules are the same as those of the corresponding embodiments mentioned above, and will not be repeated herein.
An embodiment of the present application further provides an electronic device, which has the imaging apparatus shown in FIG. 4.
Please refer to FIG. 5, and FIG. 5 is a schematic structural diagram of an elastography device according to an optional embodiment of the present application. As shown in FIG. 5, the elastography device may include at least one processor 51, for example, a CPU (Central Processing Unit, Central Processing Unit), at least one communication interface 53, a memory 54, and at least one communication bus 52. The communication bus 52 is configured to implement connection and communication between these components. The communication interface 53 may include a display screen (Display) and a keyboard (Keyboard), and optionally, the communication interface 53 may also include a standard wired interface and a standard wireless interface. The memory 54 may be a high-speed RAM memory (Random Access Memory, Volatile Random Access Memory), and may also be a non-volatile memory (non-volatile memory), such as at least one disk memory. Optionally, the memory 54 may also be at least one storage apparatus located far away from the above-mentioned processor 51. The processor 51 may be combined with the apparatus described in FIG. 4, the memory 54 stores an application program, and the processor 51 invokes the program code stored in the memory 54, to implement any one of the method steps mentioned above.

The communication bus 52 may be a peripheral component interconnect (Peripheral Component Interconnect, PCI for short) bus, or an extended industry standard architecture (extended industry standard architecture, EISA for short) bus, etc. The communication bus 52 may be classified into an address bus, a data bus, a control bus, and the like. For ease of representation, only one thick line is used in FIG. 5, but it does not indicate that there is only one bus or one type of bus.

The memory 54 may include a volatile memory (English: volatile memory), for example, a random-access memory (English: random-access memory, RAM for short). The memory may also include a non-volatile memory (English: non-volatile memory), such as a flash memory (English: flash memory), a hard disk drive (English: hard disk drive, HDD for short), or a solid-state drive (English: solid-state drive, SSD for short). The memory 54 may also include a combination of the above-mentioned types of memories.

The processor 51 may be a central processing unit (Central Processing Unit, CPU for short), a network processor (Network Processor, NP for short), or a combination of CPU and NP.

The processor 51 can further include a hardware chip. The above-mentioned hardware chip may be an application-specific integrated circuit (English: application-specific integrated circuit, ASIC for short), a programmable logic device (English: programmable logic device, PLD for short), or a combination thereof. The above-mentioned PLD may be a complex programmable logic device (English: complex programmable logic device, CPLD for short), a field-programmable gate array (English: field-programmable gate array, FPGA for short), a general array logic (English: general array logic, GAL for short), or any combination thereof.

Optionally, the memory 54 is further configured to store a program instruction, and the processor 51 may invoke the program instruction to implement the imaging method as shown in any embodiment of the present application.

The embodiments of the present application further provide a non-transitory computer storage medium, the computer storage medium stores computer-executable instructions, and the computer-executable instructions can implement the imaging method in any of the described method embodiments. The storage medium may be a magnetic disk, an optical disk, a read-only memory (Read-Only Memory, ROM), a random access memory (Random Access Memory, RAM), a flash memory, a hard disk drive (Hard Disk Drive, HDD for short), a solid-state drive (Solid-State Drive, SSD), etc. The storage medium may also include a combination of the above-mentioned types of memories.

Although the embodiments of the present application have been described in conjunction with the accompanying drawings, various modifications and variations can be made by those skilled in the art without departing from the spirit and scope of the present application, and these modifications and variations fall within the scope of protection defined by the appended claims.

### Claims

1. An elastography method, comprising:

   acquiring an elastography area of a to-be-detected tissue and a target pressure range of the to-be-detected tissue;
   determining a detection position where an elasticity detection probe is located when performing elasticity detection in the elastography area;
   performing the elasticity detection in the elastography area for a plurality of times based on the detection position and a plurality of pressure values within the target pressure range, to obtain a plurality of elastography results; and
   fusing the plurality of elastography results to determine a target elastography result of the to-be-detected tissue.

2. The method according to claim 1, wherein the performing the elasticity detection in the elastography area for the plurality of times based on the detection position and the plurality of pressure values within the target pressure range, to obtain the plurality of elastography results comprises:

acquiring a pressure value currently applied to the elastography area when the elasticity detection probe performs ultrasonic scanning at the detection position, to obtain a current pressure value; and

sequentially adjusting the current pressure value to each pressure value among the plurality of pressure values, and performing the elasticity detection in the elastography area based on the detection position and the adjusted pressure value each time, to obtain the plurality of elastography results.

3. The method according to claim 2, wherein the acquiring the current pressure value comprises:
acquiring the current pressure value through a pressure detection unit corresponding to the elasticity detection probe, wherein the pressure detection unit is configured to detect a pressure applied by the elasticity detection probe to the elastography area.

4. The method according to claim 2 or 3, wherein the performing the elasticity detection in the elastography area based on the detection position and the adjusted pressure value each time, to obtain the plurality of elastography results comprises:

performing, based on the detection position, the elasticity detection in the elastography area respectively by using the adjusted pressure value each time, to obtain a group velocity as well as a phase velocity of a shear wave at different pressure values; and

determining an elastography result corresponding to each pressure value based on the group velocity as well as the phase velocity of the shear wave at different pressure values.

5. The method according to claim 4, wherein the fusing the plurality of elastography results to determine the target elastography result of the to-be-detected tissue comprises:

determining pressure index information based on the group velocity as well as the phase velocity of the shear wave at different pressure values; and

fusing the plurality of elastography results based on the pressure index information, to determine the target elastography result.

6. The method according to claim 1, wherein the performing the elasticity detection in the elastography area for the plurality of times based on the detection position and the plurality of pressure values within the target pressure range, to obtain the plurality of elastography results comprises:

acquiring a target detection direction corresponding to the elastography area, wherein the target detection direction is a normal vector direction of the detection position;

acquiring a current detection direction of the elasticity detection probe at the detection position;

controlling the elasticity detection probe to move based on a difference between the current detection direction and the target detection direction, to adjust the current detection direction to the target detection direction; and

performing the elasticity detection in the elastography area for the plurality of times according to the adjusted current detection direction and the plurality of pressure values within the target pressure range, to obtain the plurality of elastography results.

7. The method according to claim 1, wherein the performing the elasticity detection in the elastography area for the plurality of times based on the detection position and the plurality of pressure values within the target pressure range, to obtain the plurality of elastography results comprises:

acquiring a target detection direction corresponding to the elastography area, wherein the target detection direction is a normal vector direction of the detection position;

acquiring a current detection direction of the elasticity detection probe at the detection position;

controlling the elasticity detection probe to move by a corresponding angle based on a difference between the current detection direction and the target detection direction, to adjust the current detection direction to the target detection direction;

acquiring a pressure value applied to the elastography area when the elasticity detection probe performs ultrasonic scanning at the detection position along the adjusted current detection direction, to obtain a current pressure value; and

sequentially adjusting the current pressure value to each pressure value among the plurality of pressure values, and performing the elasticity detection in the elastography area by using the adjusted pressure value each time to obtain the plurality of elastography results.

8. The method according to claim 1, wherein the performing the elasticity detection in the elastography area for the plurality of times based on the detection position and the plurality of pressure values within the target pressure range, to obtain the plurality of elastography results comprises:

determining a normal vector direction of the detection position;
acquiring a pressure value applied to the elastography area when the elasticity detection probe performs ultrasonic scanning at the detection position along the normal vector direction to obtain a current pressure value; and
sequentially adjusting the current pressure value to each pressure value among the plurality of pressure values, and performing the elasticity detection in the elastography area by using the adjusted pressure value each time, to obtain the plurality of elastography results.

9. An elastography apparatus, comprising:

an acquisition module, configured to acquire an elastography area of a to-be-detected tissue and a target pressure range of the to-be-detected tissue;
a first determination module, configured to determine a detection position where an elasticity detection probe is located when performing elasticity detection in an elastography area;
an imaging module, configured to perform the elasticity detection in the elastography area for a plurality of times based on the detection position and a plurality of pressure values within the target pressure range, to obtain a plurality of elastography results; and
a second determination module, configured to fuse the plurality of elastography results to determine a target elastography result of the to-be-detected tissue.

10. An elastography device, comprising: a memory and a processor, wherein the memory and the processor are in communication connection with each other, the memory stores computer instructions, and the processor executes the computer instructions, to implement the elastography method according to any one of claims 1-8.

11. A computer-readable storage medium, wherein the computer-readable storage medium stores computer instructions, and the computer instructions are used to enable a computer to implement the elastography method according to any one of claims 1-8.

Acquiring an elastography area of a to-be-detected tissue and a target pressure range of the to-be-detected tissue

S11

Determining a detection position where an elasticity detection probe is located when performing elasticity detection in the elastography area

S12

Performing the elasticity detection in the elastography area for a plurality of times based on the detection position and a plurality of pressure values within the target pressure range, to obtain a plurality of elastography results

S13

Fusing the plurality of elastography results to determine a target elastography result of the to-be-detected tissue

S14

FIG. 1

S21

Acquiring an elastography area of a to-be-detected tissue and a target pressure range of the to-be-detected tissue

S22

Determining a detection position where an elasticity detection probe is located when performing elasticity detection in the elastography area

S231

Acquiring a pressure value currently applied to the elastography area when the elasticity detection probe performs ultrasonic scanning at the detection position, to obtain a current pressure value

S23

S232

Sequentially adjusting the current pressure value to each pressure value among the plurality of pressure values, and performing the elasticity detection in the elastography area based on the detection position and the adjusted pressure value each time, to obtain the plurality of elastography results

S241

Determining pressure index information based on the group velocity as well as the phase velocity of the shear wave at different pressure values

S24

S242

Fusing the plurality of elastography results based on the pressure index information, to determine the target elastography result

FIG. 2

Acquiring an elastography area of a to-be-detected tissue and a target pressure range of the to-be-detected tissue — S31

Determining a detection position where an elasticity detection probe is located when performing elasticity detection in the elastography area — S32

S33

Acquiring a target detection direction corresponding to the elastography area — S331

Acquiring a current detection direction of the elasticity detection probe at the detection position — S332

Controlling the elasticity detection probe to move based on a difference between the current detection direction and the target detection direction, to adjust the current detection direction to the target detection direction — S333

Performing the elasticity detection in the elastography area for the plurality of times according to the adjusted current detection direction and the plurality of pressure values within the target pressure range, to obtain the plurality of elastography results — S334

Fusing the plurality of elastography results to determine a target elastography result of the to-be-detected tissue — S34

FIG. 3

41

Acquisition module

42

First determination
module

43

Imaging module

44

Second determination
module

FIG. 4

51

Processor

52

53

Communication
interface

54

Memory

Operating system

Application
program

FIG. 5

## INTERNATIONAL SEARCH REPORT

| International application No. |
|---|
| **PCT/CN2024/096044** |

**A.    CLASSIFICATION OF SUBJECT MATTER**

A61B8/00(2006.01)i

According to International Patent Classification (IPC) or to both national classification and IPC

**B.    FIELDS SEARCHED**

Minimum documentation searched (classification system followed by classification symbols)

IPC:  A61B

Documentation searched other than minimum documentation to the extent that such documents are included in the fields searched

Electronic data base consulted during the international search (name of data base and, where practicable, search terms used)

CNTXT: ENTXTC; VEN; ENTXT; CNKI; 超星独秀, CHAOXING; 万方, WANFANG; ISI Web of science: 何琼, 邵金华, 孙锦, 无锡海斯凯尔, 超声, 弹性, 剪切波, 声辐射力, 压力, 压缩力, 融合, 权重, AFRI, SWE, ultraso+, shear wave, elastography, acoustic radiation force, force, fuse, weight+

**C.    DOCUMENTS CONSIDERED TO BE RELEVANT**

| Category* | Citation of document, with indication, where appropriate, of the relevant passages | Relevant to claim No. |
|---|---|---|
| PX | CN 116671968 A (WUXI HISKY MEDICAL CO., LTD.) 01 September 2023 (2023-09-01) claims 1-11 | 1-11 |
| X | US 2009203997 A1 (USTUNER KUTAY) 13 August 2009 (2009-08-13) description, paragraphs [0008]-[0050], and figures 1-3 | 1-4, 6-11 |
| X | JP 2005074077 A (FUJI PHOTO FILM CO., LTD.) 24 March 2005 (2005-03-24) description, paragraphs [0013]-[0049] | 1-3, 6-11 |
| X | US 2012321165 A1 (SUDA MASAHIRO et al.) 20 December 2012 (2012-12-20) description, paragraphs [0002]-[0094] | 1-3, 6-11 |
| A | US 2009292205 A1 (OSAKA TAKASHI) 26 November 2009 (2009-11-26) entire document | 1-11 |
| A | CN 114173670 A (SHENZHEN MINDRAY BIO-MEDICAL ELECTRONICS CO., LTD.) 11 March 2022 (2022-03-11) entire document | 1-11 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **14 August 2024** | **26 August 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/096044** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 113679425 A (SHENZHEN MINDRAY BIO-MEDICAL ELECTRONICS CO., LTD. et al.) 23 November 2021 (2021-11-23) entire document | 1-11 |

Form PCT/ISA/210 (second sheet) (July 2022)

### INTERNATIONAL SEARCH REPORT
**Information on patent family members**

International application No.

**PCT/CN2024/096044**

| Patent document cited in search report | | | Publication date (day/month/year) | Patent family member(s) | | | Publication date (day/month/year) |
|---|---|---|---|---|---|---|---|
| CN | 116671968 | A | 01 September 2023 | None | | | |
| US | 2009203997 | A1 | 13 August 2009 | None | | | |
| JP | 2005074077 | A | 24 March 2005 | None | | | |
| US | 2012321165 | A1 | 20 December 2012 | US | 9310473 | B2 | 12 April 2016 |
| US | 2009292205 | A1 | 26 November 2009 | None | | | |
| CN | 114173670 | A | 11 March 2022 | None | | | |
| CN | 113679425 | A | 23 November 2021 | None | | | |

Form PCT/ISA/210 (patent family annex) (July 2022)

# EP 4 721 672 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310626184